# EUROPEAN PATENT APPLICATION

(11) **EP 4 252 774 A1**
(43) Date of publication of application: **04.10.2023**
(21) Application number: 21897214.9
(22) Date of filing: 30.11.2021
(51) Int. Cl.: A61K 45/06, A61P 35/00

(54) **COMBINATION THERAPY FOR TREATING PIK3CA-MUTATED CANCER**

(30) Priority: 30.11.2020 CN 202011379205
(71) Applicant: Adlai Nortye Biopharma Co., Ltd., Yuhang District Hangzhou Zhejiang 311121 (CN)
(72) Inventor: HE, Nanhai, Hangzhou, Zhejiang 311121 (CN); CHEN, Peng, Hangzhou, Zhejiang 311121 (CN); YU, Zhiyong, Hangzhou, Zhejiang 311121 (CN); LU, Yang, Hangzhou, Zhejiang 311121 (CN)
(74) Representative: Boult Wade Tennant LLP
(86) International application number: PCT/CN2021/134189
(87) International publication number: WO 2022/111714

(57) **Abstract**

A combination therapy for treating PIK3CA-mutated cancer, comprising administering a PI3K inhibitor, a prostaglandin receptor (PGE2) inhibitor, and a PD-1 inhibitor to a subject in need thereof. The anti-tumor activity brought about by a three-drug combination method is significantly superior to that brought about by a two-drug combination method of the PI3K inhibitor and the PD-1 inhibitor, and the prostaglandin receptor (PGE2) and the PD-1 inhibitor.

## Description

This application claims priority to a Chinese patent application with application number 202011379205.7 and titled "COMBINATION THERAPY FOR TREATING PIK3CA-MUTATED CANCER" filed with the China Patent Office on November 30, 2020, the entire contents of which are incorporated herein by reference.

### Technical Field

The present invention relates to a combined treatment method and pharmaceutical composition and/or pharmaceutical combination, comprising administering a PI3K inhibitor, a prostaglandin receptor (PGE2) inhibitor and a PD-1 inhibitor to a subject in need.

### Background

Immune checkpoint inhibitors (ICIs) using anti-CTLA4 antibodies and anti-PD(L)1 antibodies have brought profound changes to cancer immunotherapy. Since the first immune checkpoint inhibitor (ICI) was approved in 2001, this series of drugs has attracted widespread attention due to their durable clinical effects in complex cancers. However, there are still a large number of cancer patients who do not respond to ICI therapy. So far, the response rate of cancer patients treated with ICI is about 20%, so it is very necessary to find a more effective way of immunotherapy to further use the immune system to treat cancer.

### Pan-type I P13K inhibitors

The dysregulation of PI3K-AKT-mTOR signaling pathway has a high incidence in cancer patients. It mainly regulates key cellular processes such as cell growth, proliferation, and metabolism, and can promote the survival, expansion and dissemination of cancer cells in cancer patients. The PI3K isoform p110α encoded by the PIK3CA gene is the second most mutated proto-oncogene in human cancer.

In the PI3K family, PI3Kβ is also involved in tumorigenesis, but PI3Kα plays a major role. In addition, the other two isoforms, PI3Kγ and PI3Kδ, play an important role in regulating the immune system. PI3Kδ can regulate and maintain the function of regulatory T cells (Treg), and PI3Kγ can not only recruit suppressive myeloid cells into the cancer microenvironment, but also enhance their ability to inhibit the anti-cancer effect of T cells.

The compound of formula I structure (AN2025) and its pharmaceutically acceptable salt are oral pan-I PI3K inhibitors, which are currently in the third phase of clinical research and development, and its indication is head and neck squamous cell carcinoma. It can not only inhibit wild-type PI3α, but also inhibit mutant P13Kα, and the mutation sites include (H1047R, E542K and E545K ). At the same time, it also has inhibitory activity against other PI3K **isoform**s (PI3Kβ, PI3Kγ, PI3Kδ). AN2025 has been clinically confirmed that it is effective for the occurrence of breast cancer caused by PI3K/PIK3CA mutations, and the patients with the highest response rate all have PIK3CA mutations (Campone M et al. Eur J Cancer. 2018;103:147-154) . AN2025 can also downregulate Tregs and suppressive myeloid cell function and their ability to enter the tumor microenvironment by inhibiting PI3Kδ and PI3Kγ (O'Donnell JS et al. Semin Cancer Biol. 2018;48:91-103. Borazanci et al. The Oncol. 2020).

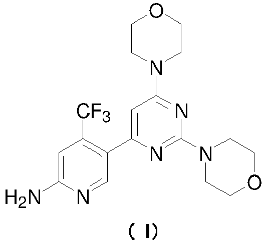

### EP-4 inhibitor

In cancer, the signaling pathway composed of prostaglandin E2 (PGE2) and its subtype 4 receptor (EP-4) can not only promote the occurrence of cancer, but also form a cancer microenvironment that promotes cancer development and immunosuppression. The cAMP produced by the activation of the PGE2/EP-4 signaling pathway in T cells can activate the cAMP-PKA signaling pathway, and this process can effectively inhibit the function of T cells. The PGE2/EP-4 signaling pathway can also negatively regulate DC maturation and induce the formation of various immunosuppressive cells including M2 macrophages and myeloid suppressor cells (MDSC) (Tomić S et al. Front Immunol. 2019; 10:475).

The compound (AN0025) with the structure of formula (II) and the pharmaceutically acceptable salt thereof is a highly selective small molecule inhibitor against E-type prostaglandin receptor 4. In preclinical studies, AN0025 can effectively reduce immunosuppressive cells in the tumor microenvironment by inhibiting the PGE2/EP-4 signaling pathway. AN0025 has been implemented in multiple clinical strategies. In phase I clinical trials of FIH (first in human) for a variety of advanced solid tumors, AN0025 has shown a certain single-drug effect; and in the neoadjuvant treatment of colorectal cancer, AN0025 combined with radiotherapy and chemotherapy has shown strong clinical efficacy (Wyrwicz et.al, Poster # 540, ESMO2019). At present, AN0025 and anti-PD-1 antibody (pembrolizumab) have entered clinical stage 1b/2 in multiple solid tumors (NCT04432857). Preclinical and early clinical data support AN0025 as an immunomodulator against the immunosuppressive cancer microenvironment by regulating M2 macrophages, MDSCs and T cells.

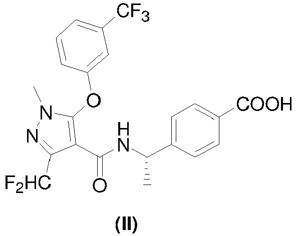

### PD-(L) 1 antibody

The PD-1 and PD-L1 signaling pathways play a key role in regulating T cell activation. PD-L1 is mainly expressed on immune cells as well as most human cancer cells. In the tumor microenvironment, cancer PD-L1 interacts with PD-1 on T cells, ultimately inhibiting the cancer cell-killing activity of T cells. A large number of successful cases of anti-PD-(L)1 therapy make it the cornerstone of modern tumor immunotherapy.

AN2025 and AN0025 can systemically modulate the immunosuppressive tumor microenvironment, which is complementary to the mechanism by which anti-PD(L)1 therapy kills cancer. Targeting PIK3CA mutations with AN2025 can add additional mechanisms of action, allowing patients to benefit from the immunomodulatory activity of this triple combination. This triple combination provides an ideal treatment option for most patients with advanced solid tumors, especially those with PIK3CA mutations.

### Contents of the invention

The technical problem to be solved by the present invention is to solve the problem of low response rate of cancer patients treated with ICI in the prior art. The purpose of the present invention is to provide a more effective way of immunotherapy to further utilize the immune system to treat cancer.

In view of this, the present invention provides a method for treating cancer by combining a PI3K inhibitor, a prostaglandin receptor (PGE2) inhibitor and a PD-1 inhibitor, and the antitumor activity of the method is significantly better than that of any **pairwise** combination.

Specifically, the present invention provides the following technical solutions:
A method for treating tumor/cancer and/or generating a memory immune response against tumor/cancer in a subject in need thereof, comprising administering to said subject an effective amount of a PI3K inhibitor, a prostaglandin receptor (PGE2) inhibitor, and a PD-1 inhibitor, wherein the PI3K inhibitor, the prostaglandin receptor (PGE2) inhibitor and the PD-1 inhibitor can be administered simultaneously, separately or sequentially as a single **dosage form.**

In one embodiment of the present invention, wherein the PI3K inhibitor is selected from PI3Kα, PI3Kβ, PI3Kγ, PI3Kδ subtype inhibitors.

In one embodiment of the present invention, wherein the PI3K inhibitor is selected from Idelalisib, Copanlisib, Duvelisib, Alpelisib, Seletalisib, Gedatolisib, Rigosertib sodium, Leniolisib, Umbralisib, Buparlisib (AN2025), AMG-319, GM-604, Acalisib , Bimiralisib, GDC-0084, ACP-319, Tenalisib, serabelisib, SF-1126, Nemiralisib, Fimepinostat, LY-3023414, Voxtalisib, Dactolisib, Parsaclisib, GSK-2636771, AZD-8186, ASN-003, or any combination thereof.

In one embodiment of the present invention, wherein the PI3K inhibitor is a compound of formula (I) or a pharmaceutically acceptable salt thereof,

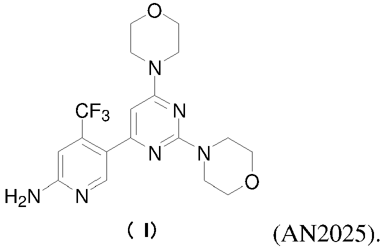

In one embodiment of the present invention, wherein said prostaglandin receptor (PGE2) inhibitor is selected from EP-1, EP-2, EP-3 and EP-4 inhibitors.

In one embodiment of the present invention, wherein the EP-4 inhibitor is selected from compounds having formula (II) or pharmaceutically acceptable salts thereof.

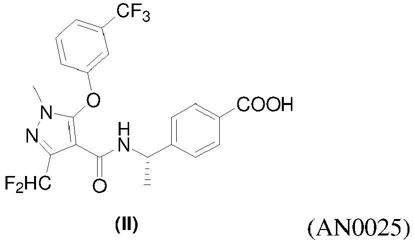

In one embodiment of the present invention, the PD-1 inhibitor includes PD-1/PD-L1 antibody therapy.

In one embodiment of the present invention, wherein the PD-1 inhibitor is selected from: PD-1 antibody, PD-L1 antibody and PD-L2 antibody; preferably, wherein the PD-1 inhibitor is selected from Nivolumab, Pembrolizumab, Atezolizumab, Avelumab, Durvalumab, Tremelimumab, **Toripalimumab, Sintilimab, Tislelizumab, Camrelizumab,** or any combination thereof.

In one embodiment of the present invention, wherein the PI3K inhibitor is administered in a dosage form of oral, buccal or parenteral route, for example, the dosage form of the oral route may be tablet, capsule, powder, pill, granule, suspensions, solutions and solution preconcentrates, emulsions and emulsion preconcentrates, for example, dosage forms for the parenteral route may be **those** for intravenous, intraperitoneal, intradermal, subcutaneous, intramuscular, intracranial, intrathecal, intratumoral, transdermal and transmucosal administration.

In one embodiment of the present invention, wherein the PI3K inhibitor is administered once or twice a day; or the PI3K inhibitor is administered once every 2, 3, 4, 5, 6, 7, 8, 9, 10 days or every 1, 2 or 3 weeks; or the PI3K inhibitor is administered once a day for 5 consecutive days a week, followed by an interval of 2 days.

In one embodiment of the present invention, wherein said PI3K inhibitor is administered in a **dose range** of about 20 mg/day to about 200 mg/day, about 30 mg/day to about 160 mg/day, about 60 mg/day to about 120 mg/day in adults.

In the technical solution of the present invention, wherein the PI3K inhibitor is administered to the subject at an effective dose of about 0.5 to about 250mg/kg, 1 to about 250mg/kg, about 2 to about 200mg/kg, about 3 to about 120mg/kg, about 5 to about 250mg/kg, about 10 to about 200 mg/kg, or about 20 to about 120 mg/kg.

In one embodiment of the present invention, wherein the prostaglandin receptor (PGE2) inhibitor is administered orally, buccally or parenteral route, for example, the dosage form of the oral route can be tablets, capsules, powders, pills, granules, suspensions, solutions and solution preconcentrates, emulsions and emulsion preconcentrates, e.g. dosage forms for the parenteral route can be dosage forms for intravenous, intraperitoneal, intradermal, subcutaneous, intramuscular, intracranial, intrathecal, intratumoral, intratumoral, transdermal, and transmucosal administration.

In one embodiment of the present invention, wherein the prostaglandin receptor (PGE2) inhibitor is administered once or twice a day; or the prostaglandin receptor (PGE2) inhibitor is administered once every 2, 3, 4, 5, 6, 7, 8, 9, 10 days or every 1, 2 or 3 weeks; or the prostaglandin receptor (PGE2) inhibitor is administered once a day for 5 consecutive days a week, followed by an interval of 2 days.

In one embodiment of the present invention, wherein said prostaglandin receptor (PGE2) is administered in a dose range of about 20mg/day-about 2000mg/day, about 30mg/day-about 1600mg/day, about 60mg/day-about Dosage ranges of 1200 mg/day, about 100 mg/day to about 1000 mg/day in an adult.

In one embodiment of the present invention, wherein the prostaglandin receptor (PGE2) inhibitor is administered to the subject at an effective dose of about 0.5 to about 250 mg/kg, 1 to about 250 mg/kg, about 2 to about 200 mg/kg, about 3 to about 120 mg/kg, about 5 to about 250 mg/kg, about 10 to about 200 mg/kg, or about 20 to about 120 mg/kg.

In one embodiment of the present invention, wherein the PD-1 inhibitor is administered by an extradigestive route, for example, the extradigestive route may be intravenous, intraperitoneal, intradermal, subcutaneous, intramuscular, intracranial, intrathecal, intratumoral, percutaneous penetration, transmucosal administration.

In one embodiment of the present invention, the PD-1 inhibitor is formulated into a solution, a lyophilized agent, or a powder injection.

In one embodiment of the present invention, wherein the PD-1 inhibitor is administered at an effective dose of 0.05mg/kg, 0.1mg/kg, 1mg/kg, 2mg/kg, 3mg/kg, 4mg/kg, 5mg/kg, 6mg/kg, 7mg/kg or 8mg/kg.

In one embodiment of the present invention, wherein said tumor/cancer is selected from PIK3CA mutated cancers.

In one embodiment of the invention, wherein said tumor/cancer is selected from the group consisting of: head and neck squamous cell carcinoma, head and neck cancer, brain cancer, glioma, glioblastoma multiforme, neuroblastoma Cell tumor, central nervous system cancer, neuroendocrine tumor, throat cancer, nasopharyngeal cancer, esophageal cancer, thyroid cancer, malignant pleural mesothelioma, lung cancer, breast cancer, liver cancer, hepatoma, hepatobiliary cancer, pancreatic cancer, gastric cancer, gastrointestinal cancer, bowel cancer, colon cancer, colorectal cancer, kidney cancer, clear cell renal cell carcinoma, ovarian cancer, endometrial cancer, cervical cancer, bladder cancer, prostate cancer, testicular cancer, skin cancer, melanoma , leukemia, lymphoma, bone cancer, chondrosarcoma, myeloma, multiple myeloma, myelodysplastic syndrome, myeloproliferative neoplasms, squamous cell carcinoma, Ewing's sarcoma, systemic light chain amyloidosis and Kerr cell carcinoma; more preferably, the lymphoma is selected from: Hodgkin's lymphoma, non-Hodgkin's lymphoma, diffuse large B-cell lymphoma, follicular lymphoma, primary mediastinal large B - cell lymphoma, mantle cell lymphoma, small lymphocytic lymphoma, T-cell/histiocytic rich large B-cell lymphoma and lymphoplasmacytic lymphoma, said lung cancer being selected from the group consisting of: non-small cell lung cancer and small cell lung cancer selected from the group consisting of chronic myeloid leukemia, acute myeloid leukemia, lymphocytic leukemia, lymphoblastic leukemia, acute lymphoblastic leukemia, chronic lymphocytic leukemia and myeloid leukemia.

In addition, the present invention also provides a pharmaceutical composition and/or pharmaceutical combination, which comprises a PI3K inhibitor, a prostaglandin receptor (PGE2) inhibitor and a PD-1 inhibitor, and a pharmaceutically acceptable carrier.

In the pharmaceutical composition and/or pharmaceutical combination of the present invention, the PI3K inhibitor is selected from PI3Kα, PI3Kβ, PI3Kγ, and PI3Kδ subtype inhibitors.

In the pharmaceutical composition and/or pharmaceutical combination of the present invention, wherein the PI3K inhibitor is selected from Idelalisib, Copanlisib, Duvelisib, Alpelisib, Seletalisib, Gedatolisib, Rigosertib sodium, Leniolisib, Umbralisib, Buparlisib (AN2025), AMG-319, or any combination thereof.

In the pharmaceutical composition and/or pharmaceutical combination of the present invention, wherein the PI3K inhibitor is a compound of formula (I) or a pharmaceutically acceptable salt thereof,

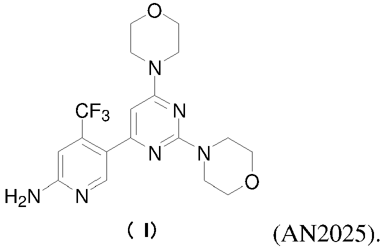

In the pharmaceutical composition and/or pharmaceutical combination of the present invention, wherein the prostaglandin receptor (PGE2) inhibitor is selected from EP-1, EP-2, EP-3 and EP-4 inhibitors.

In the pharmaceutical composition and/or pharmaceutical combination of the present invention, the EP-4 inhibitor is selected from compounds of formula (II) or pharmaceutically acceptable salts thereof.

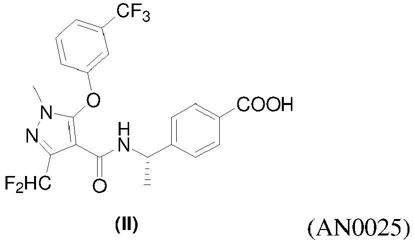

In the pharmaceutical composition and/or pharmaceutical combination of the present invention, wherein the PD-1 inhibitor is selected from: PD-1 antibody, PD-L1 antibody and PD-L2 antibody; preferably, wherein the PD-1 inhibitor is selected from Nivolumab, Pembrolizumab, Atezolizumab, Avelumab, Durvalumab, Tremelimumab, Toripalimab, Sintilimumab, Tislelizumab, Camrelizumab or any combination thereof.

In the pharmaceutical composition and/or pharmaceutical combination of the present invention, wherein the PI3K inhibitor, prostaglandin receptor (PGE2) inhibitor and PD-1 inhibitor can be in the same and/or separate dosage form (dosage form) .

In the pharmaceutical composition and/or pharmaceutical combination of the present invention, wherein the pharmaceutical composition and/or pharmaceutical combination can be **formulated** into a dosage form by oral, buccal or parenteral route, for example, the dosage form of the oral route can be tablets, capsules, powders, pills, granules, suspensions, solutions and solution pre-concentrates, emulsions and emulsion pre-concentrates, for example the dosage form for the parenteral route may be intravenous, intraperitoneal, intradermal, subcutaneous, intramuscular, intracranial, intrathecal, intratumoral, percutaneous penetration, and transmucosal administration. The dosage forms can be for example, solution, powder injection or freeze-dried preparation.

In the pharmaceutical composition and/or pharmaceutical combination of the present invention, the PI3K inhibitor can be formulated into a dosage form that is via oral, buccal, or parenteral route, for example, the dosage form of the oral route can be tablets, capsules, Powders, pills, granules, suspensions, solutions and solution preconcentrates, emulsions and emulsion preconcentrates, e.g. dosage forms for the parenteral route can be intravenous, intraperitoneal, intradermal, subcutaneous, intramuscular, intracranial, sheath The dosage forms for intratumoral, transdermal and transmucosal administration. The dosage form can be, for example, solution, powder injection or lyophilized preparation.

In the pharmaceutical composition and/or pharmaceutical combination of the present invention, wherein the prostaglandin receptor (PGE2) inhibitor can be formulated into a dosage form by oral, buccal or parenteral route, such as the dosage form of the oral route Can be tablets, capsules, powders, pills, granules, suspensions, solutions and solution preconcentrates, emulsions and emulsion preconcentrates, for example the dosage form for the parenteral route can be intravenous, intraperitoneal, intradermal, subcutaneous , intramuscular, intracranial, intrathecal, intratumoral, transdermal and transmucosal administration. The dosage form, for example, may be solution, powder injection or lyophilized preparation.

In the pharmaceutical composition and/or drug combination of the present invention, the PD-1 inhibitor can be formulated into a dosage form administered by a parenteral route, for example, the parenteral route can be intravenous, intraperitoneal, intradermal, Subcutaneous, intramuscular, intracranial, intrathecal, intratumoral, transdermal, transmucosal administration. The dosage form, for example, can be solution, powder injection or freeze-dried preparation.

In the pharmaceutical composition and/or pharmaceutical combination of the present invention, wherein each unit dosage form (unit dosage form) contains 1-1000 mg dose of PI3K inhibitor, such as 1, 2, 3, 4, 5, 6, 7, 8 , 9, 10, 11, 12, 13, 14, 15, 16, 17, 18, 19, 20, 25, 30, 35, 40, 50, 60, 70, 75, 80, 90, 100, 110, 120 , 125, 130, 140, 150, 160, 170, 175, 180, 190, 200, 250, 300, 350, 400, 450, 500, 600, 700, 750, 800, 900, 1000mg or a value between any two of the above values.

In the pharmaceutical composition and/or pharmaceutical combination of the present invention, wherein each unit dosage form (unit dosage form) contains a prostaglandin receptor (PGE2) inhibitor in a dose of 1-1000 mg, such as 1, 2, 3, 4, 5 , 6, 7, 8, 9, 10, 11, 12, 13, 14, 15, 16, 17, 18, 19, 20, 25, 30, 35, 40, 50, 60, 70, 75, 80, 90, 100, 110, 120, 125, 130, 140, 150, 160, 170, 175, 180, 190, 200, 250, 300, 350, 400, 450, 500, 600, 700, 750, 800, 900, 1000mg or a value between any two of the above values.

In the pharmaceutical composition and/or pharmaceutical combination of the present invention, each unit dosage form contains a dose of 1-5000 mg of PD-1 inhibitors, such as 1, 2, 3, 4, 5, 6, 7, 8, 9, 10, 11, 12, 13, 14, 15, 16, 17, 18, 19, 20, 25, 30, 35, 40, 50, 60, 70, 75, 80, 90, 100, 110, 120, 125, 130, 140, 150, 160, 170, 175, 180, 190, 200, 250, 300, 350, 400, 450, 500, 600, 700, 750, 800, 900, 1000, 1100, 1200, 1300, 1400, 1500, 1600, 1700, 1800, 1900, 2000, 2200, 2400, 2500, 2600, 2700, 2750, 2800, 3000, 3500, 4000, 4500, 5000 mg or a value between any two of the above values.

Besides, the present invention also provides a pharmaceutical composition and/or a pharmaceutical combination for treating tumor/cancer and/or generating memory immune response against tumor/cancer.

In addition, the present invention also provides the use of the pharmaceutical composition and/or pharmaceutical combination for preparing a medicine for treating tumor/cancer and/or generating memory immune response against tumor/cancer.

In addition, the present invention also provides a kit, which includes the pharmaceutical composition and/or pharmaceutical combination described in the present invention, and instructions for use, wherein the PI3K inhibitor, prostaglandin receptor (PGE2) inhibitor and the PD-1 inhibitor can be in the same and/or separate containers.

### Description of drawings

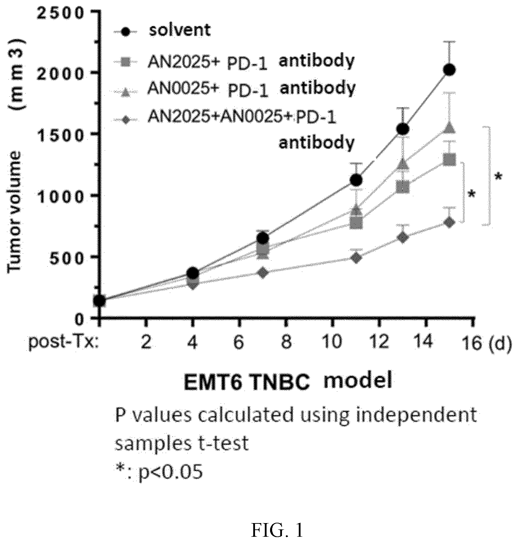

Figure 1. Tumor growth curves of individual EMT6 tumors treated with different drug combinations of AN2025, AN0025, and PD-1 antibody. 8 mice per group. AN2025 administration: 30mg/kg, orally (PO) once a day. AN0025 administration: 150mg/kg, orally (PO) once a day. PD-1 antibody administration: 10mg/kg, intraperitoneal injection (IP) twice a week.

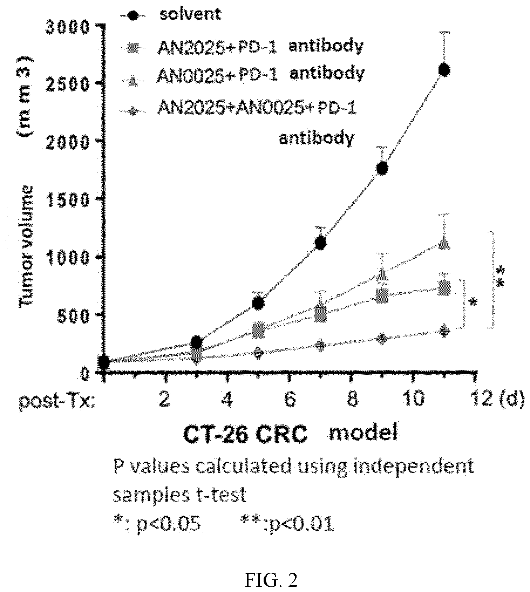

Figure 2. Tumor growth curves of individual CT-26 tumors treated with different drug combinations of AN2025, AN0025, and PD-1 antibody. 8 mice per group. AN2025 administration: 30mg/kg, orally (PO) once a day. AN0025 administration: 100mg/kg, orally (PO) once a day. PD-1 antibody administration: 10mg/kg, intraperitoneal injection (IP) twice a week.

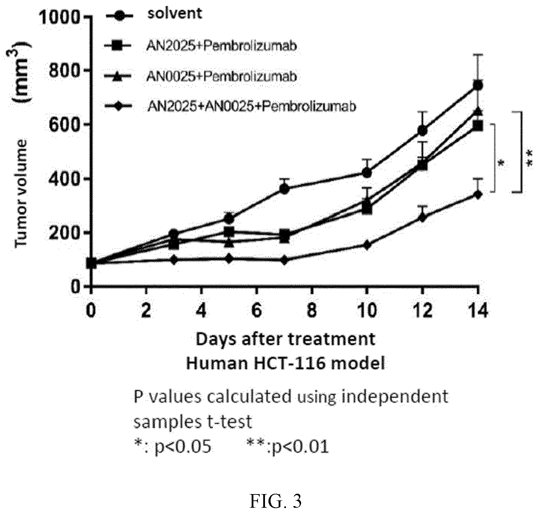

Figure 3. The growth curve of PIK3CA mutant tumor HCT116 treated with different drug combinations of AN2025, AN0025 and PD-1 antibody on humanized mice. AN2025 administration: 37.5mg/kg, orally (PO) once a day. AN0025 administration: 30mg/kg, orally (PO) once a day. PD-1 antibody administration: 10mg/kg, intraperitoneal injection (IP) twice a week.

### Embodiments

### Definition of Terms:

Unless otherwise indicated herein, terms used herein have their ordinary meanings in the art.

The term "PI3K inhibitor" herein refers to phosphatidylinositol 3-kinase, which is a highly conserved enzyme family and an important part of the intracellular PI3K-Akt-mTOR signaling axis. Examples of PI3K inhibitors include, but are not limited to, inhibitors of PI3Kα, PI3Kβ, PI3Kγ, PI3Kδ subtypes. Specific examples include but are not limited to Idelalisib, Copanlisib, Duvelisib, Alpelisib, Seletalisib, Gedatolisib, Rigosertib sodium, Leniolisib, Umbralisib, Buparlisib (AN2025), AMG-319, GM-604, Acalisib, Bimiralisib, GDC-0084, ACP- 319, Tenalisib, serabelisib, SF-1126, Nemiralisib, Fimepinostat, LY-3023414, Voxtalisib, Dactolisib, Parsaclisib, GSK-2636771, AZD-8186, ASN-003 and any combination thereof. Preferred examples include but are not limited to Compounds of formula I as taught herein (also referred to herein as AN2025) and pharmaceutically acceptable salts thereof.

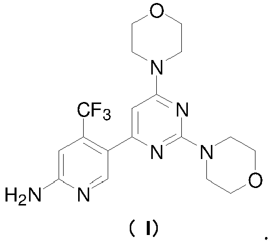

The term "EP-4 inhibitory agent" refers herein to a compound that inhibits or blocks cellular signaling triggered by the interaction of PGE2 with the EP-4 receptor. Examples of EP-4 inhibitors include, but are not limited to, ER-819762, MK-2894, MF498, ONO-AE3-208, Evatanepag, ONO-AE2-227, CJ-042794, EP4A, BGC201531, CJ-023423, ONO-AE3 -240, GW627368 and AH23848, for example listed in the IUPHAR database as inhibitors of the EP-4 receptor. Other examples include, but are not limited to, compounds of Formula II as taught herein and pharmaceutically acceptable salts thereof (also referred to herein as AN0025).

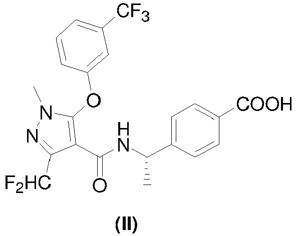

In this article, the term "PD-1 inhibitor" has the same meaning as "PD-1 and/or PD-L1 antibody", which refers to the anti-programmed death protein 1 (PD-1) / programmed death protein ligand 1 (PD-L1) antibody. Exemplary antibodies include, but are not limited to, those shown in Patent Nos. US7,029,674, US7,488,802, US7,521,051, US8,008,449, US8,354,509, US8,617,546, and US8,709,417. Specific examples of PD-1/PD-L1 inhibitors also include Nivolumab, Pembrolizumab, Atezolizumab, Avelumab, Durvalumab, Tremelimumab, Toripalimab, Sintilimab, Tislelizumab, Camrelizumab monoclonal antibody or any combination thereof.

"Treating", "treating" and "treating" refer to alleviating, inhibiting and/or reversing the progression of a disease (eg, tumor/cancer) in a subject in need thereof. The term "treatment" includes any indication of successful treatment or amelioration of a disease, including any objective or subjective parameter, such as alleviation; palliation; reduction of symptoms or making the injury, pathology or condition more tolerant to the subject; delay or slowing of the rate of progression, and the like. Measures of treatment or improvement may be based, for example, on the results of physical, pathological, and/or diagnostic tests known in the art. For example, in one embodiment, the "treatment" of cancer involved in the present invention refers to the combination therapy of the PI3K inhibitor, PGE2 receptor inhibitor and PD-1 inhibitor of the present invention on cancer or a subject diagnosed with cancer, to achieve at least one positive therapeutic outcome, such as a decrease in the number of cancer cells, a decrease in tumor size, a decrease in the rate at which tumor cells infiltrate peripheral organs, or a decrease in the rate of tumor metastasis or tumor growth.

Treating can also refer to reducing the incidence or risk of onset of a disease, or reducing (eg, prolonging the time to relapse) a disease recurrence, compared to what would occur if the measure were not taken. In the medical field, this treatment is also known as "prophylaxis".

The term "effective amount" or "therapeutically effective amount" refers to an amount effective to treat a disease, as documented by clinical testing and evaluation, patient observation, and the like. An "effective amount" can further mean an amount that causes a detectable change in biological or chemical activity. A detectable change can be detected and/or further quantified by one skilled in the art familiar with the relevant mechanism or method. Additionally, an "effective amount" can mean an amount that maintains a desired physiological state (ie, reduces or prevents significant decline and/or promotes amelioration of a condition). An "effective amount" can further refer to a therapeutically effective amount.

In some embodiments, the PI3K inhibitor is administered to a subject in an effective amount. An effective amount is usually 0.01 mg/kg body weight to 500 mg/kg body weight per day. In some embodiments, the pharmaceutically acceptable compositions can be formulated such that daily doses of compounds from 0.01 mg/kg body weight to 200 mg/kg body weight or from 0.01 mg/kg body weight to 100 mg/kg body weight can be administered to subjects receiving these compositions.(eg 0.75 mg to 7.5 g or 15 g based on a 75 kg human). In certain embodiments, the active pharmaceutical ingredient of the invention is formulated to provide a dose of 0.01 mg/kg to 70 mg/kg (eg, a dose of 0.75 mg to 5.25 g based on a 75 kg human).

In some embodiments, the effective dosage of PI3K inhibitor is about 0.5 to about 250 mg/kg, 1 to about 250 mg/kg, about 2 to about 200 mg/kg, about 3 to about 120 mg/kg, about 5 to about 250 mg/kg kg, about 10 to about 200 mg/kg, or about 20 to about 120 mg/kg. In some embodiments, effective **doses** include about 0.5 mg/kg, 1 mg/kg, 2 mg/kg, 3 mg/kg, 4 mg/kg, 5 mg/kg, 6 mg/kg, 8 mg/kg, 10 mg/kg, 20 mg/kg , 25mg/kg, 40mg/kg, 50mg/kg, 60mg/kg, 75mg/kg, 100mg/kg, 120mg/kg, 150mg/kg, 175mg/kg, 200mg/kg, 225mg/kg, 250mg/kg and 300mg /kg. The dosage form may take various suitable forms, such as tablets or capsules, and the effective dose may be presented in one or more unit dosage forms (such as tablets, capsules) and provided 1, 2 or 3 times a day, or in doses such as 4 , 8 or 12 hour intervals are available throughout the day. A tablet or capsule may contain, for example, 10, 25, 50, 75, 100, 150, 200, 250, 300, 350, 400, 450, 500, 600, 700, 800, 900, 1,000, 1,100 or 1,250 mg of compound. For example, in some embodiments, administering a PI3K inhibitor to a human subject can include a daily dose of the PI3K inhibitor in the range of 100-1,250, 150-1,000, 200-800, or 250-750 mg, the daily dose being It can be administered once a day in its entirety, or in multiple portions at regular intervals throughout the day. Liquid **preparation**s can also be prepared to allow easy and convenient dispensing of any dosage.

In some embodiments, an EP-4 inhibitor is administered to a subject in an effective amount. An effective amount is usually 0.01 mg/kg body weight to 500 mg/kg body weight per day. In some embodiments, the pharmaceutically acceptable compositions can be formulated such that daily doses of compounds from 0.01 mg/kg body weight to 200 mg/kg body weight or from 0.01 mg/kg body weight to 100 mg/kg body weight can be administered to subjects receiving these compositions.(eg 0.75 mg to 7.5 g or 15 g based on a 75 kg human). In certain embodiments, the active pharmaceutical ingredient of the invention is formulated to provide a dose of 0.01 mg/kg to 70 mg/kg (eg, a dose of 0.75 mg to 5.25 g based on a 75 kg human).

In some embodiments, the effective doses of EP-4 inhibitor are about 0.5 to about 250 mg/kg, 1 to about 250 mg/kg, about 2 to about 200 mg/kg, about 3 to about 120 mg/kg, about 5 to about 250 mg/kg, about 10 to about 200 mg/kg, or about 20 to about 120 mg/kg. In some embodiments, effective **doses** include about 0.5 mg/kg, 1 mg/kg, 2 mg/kg, 3 mg/kg, 4 mg/kg, 5 mg/kg, 6 mg/kg, 8 mg/kg, 10 mg/kg, 20 mg/kg , 25mg/kg, 40mg/kg, 50mg/kg, 60mg/kg, 75mg/kg, 100mg/kg, 120mg/kg, 150mg/kg, 175mg/kg, 200mg/kg, 225mg/kg, 250mg/kg and 300mg /kg. The dosage form may take various suitable forms, such as tablets or capsules, and the effective dose may be presented in one or more unit dosage forms (such as tablets, capsules) and provided 1, 2 or 3 times a day, or in doses such as 4 , 8 or 12 hour intervals are available throughout the day. A tablet or capsule may contain, for example, 10, 25, 50, 75, 100, 150, 200, 250, 300, 350, 400, 450, 500, 600, 700, 800, 900, 1,000, 1,100 or 1,250 mg of compound. For example, in some embodiments administering an EP-4 inhibitor to a human subject can include a daily dose of the EP-4 inhibitor in the range of 100-1,250, 150-1,000, 200-800, or 250-750 mg, so the daily dose may be administered in its entirety once a day, or in divided portions at regular intervals throughout the day. Liquid preparations can also be prepared to allow easy and convenient dispensing of any dosage.

Antibodies will generally be mixed with a pharmaceutically acceptable non-toxic carrier substance (such as physiological saline or phosphate buffered saline) prior to administration, and may be administered using any medically appropriate procedure, including, for example, but not limited to intravenous or intraarterial administration and injection into cerebrospinal fluid. In some cases, intraperitoneal, intradermal, intracavity, intrathecal, or direct administration to the tumor or an artery supplying the tumor may be advantageous.

In some embodiments, the effective dosage of the antibody is about 5 to about 250 mg/kg, about 10 to about 200 mg/kg, or about 20 to about 120 mg/kg. In some embodiments, effective **doses** include 5 mg/kg, 10 mg/kg, 20 mg/kg, 25 mg/kg, 40 mg/kg, 50 mg/kg, 60 mg/kg, 75 mg/kg, 100 mg/kg, 120 mg/kg, 150 mg /kg, 175mg/kg, 200mg/kg, 225mg/kg, 250mg/kg and 300mg/kg. Dosage forms may take the form of, for example, tablets or capsules, and an effective dose may be presented in one or more tablets, capsules, etc., and provided once a day or at intervals of, for example, 4, 8 or 12 hours throughout the day. A tablet or capsule may contain, for example, 10, 25, 50, 75, 100, 150, 200, 250, 300, 350, 400, 450, 500, 600, 700, 800, 900 or 1,000 mg of antibody. Liquid preparations can also be prepared to allow easy and convenient dispensing of any dosage.

In some embodiments, the antibody is administered to a subject in an effective amount. An effective amount is usually 0.01 mg/kg to 500 mg/kg body weight per day. In some embodiments, the pharmaceutically acceptable compositions can be formulated so that daily doses of compounds from 0.01 mg/kg body weight to 200 mg/kg body weight or from 0.01 mg/kg body weight to 100 mg/kg body weight per day can be administered to patients receiving these compositions (for example a dose of 0.75 mg to 7.5 g or 15 g based on a 75 kg human). In certain embodiments, compositions of the invention are formulated to provide a dose of 0.01 mg/kg to 70 mg/kg (eg, a dose of 0.75 mg to 5.25 g based on a 75 kg human).

An effective amount of the antibody can be, for example, 0.05 mg/kg, 0.1 mg/kg, 1 mg/kg, 2 mg/kg, 3 mg/kg, 4 mg/kg, 5 mg/kg, 6 mg/kg, 7 mg/kg or 8 mg/kg per dose (eg doses of 3.75 mg to 600 mg based on a 75 kg human).

Doses of the antibody may be administered once, twice, three, four, five or more times, weekly, every two weeks, or even every three weeks during the course of treatment. The dosing time can be once a day, once every two days, once every three days, once every four days, once every five days, once a week, once every two weeks or once every three weeks. **Formulations** comprising antibodies can be **prepared** such that any **dosage** can be dispensed easily and conveniently.

The term "subject" refers to a mammalian subject, and especially a human subject, including male or female subjects, and including neonatal, infant, toddler, juvenile, adult or elderly subjects, and further includes various races and ethnicities, such as Caucasian, African and Asian. Herein, the subject has a tumor/cancer. In one embodiment, it is not considered whether the tumor patient expresses PD-L1. In another embodiment, the tumor patient is a tumor patient with positive expression of PD-L1.

The term "pharmaceutically acceptable salt" refers to a relatively non-toxic inorganic or organic acid salt of a compound of formula I or formula II of the present invention. These salts can be prepared in situ during the final isolation and purification of the compounds, or by reacting the purified compound in free form with a suitable organic or inorganic acid, respectively, and isolating the salt thus formed. Representative acid salts include (but are not limited to) acetate, adipate, aspartate, benzoate, benzenesulfonate, bicarbonate/carbonate, bisulfate/sulfate , borate, D-camphorsulfonate, citrate, cyclamate, ethanedisulfonate, ethanesulfonate, formate, fumarate, glucoheptonate, gluconate, glucuronate, hexafluorophosphate, seabenzoate, hydrochloride/chloride, hydrobromide/bromide, hydroiodide/iodide, isethionate, lactate, malate, maleate, malonate, methanesulfonate, methylsulfate, naphthylate, 2-naphthalenesulfonate, nicotinate, nitrate, orotate, oxalate, palmitate, pamoate, phosphate/hydrogen phosphate/dihydrogen phosphate, pyroglutamate, saccharate, stearate, succinate, tannate, tartrate, tosylate, trifluoroacetate and xinafoate. In one embodiment, the pharmaceutically acceptable salt is a hydrochloride/chloride salt.

The terms "tumor" and "cancer" are used interchangeably herein to refer to an uncontrolled abnormal growth of a tissue in a localized area of the body. Without timely medical intervention, the tumor/cancer can grow uncontrollably and potentially metastasize to other locations in the body, eventually killing the body.

The term "cancer" may include cancers caused by genetically inherited mutations. Examples of such cancers include, but are not limited to, breast cancer, cancers that may be associated with Li-Fraumeni syndrome (such as childhood sarcoma, leukemia, and brain cancer), cancers that may be associated with Lynch syndrome such as colon cancer, bile duct cancer, brain cancer, endometrial cancer, renal cancer, ovarian cancer, pancreatic cancer, small bowel cancer, gastric and ureteral cancer, lung cancer, melanoma, prostate cancer, retinoblastoma, thyroid cancer and uterine cancer.

In addition, cancer can be the result of acquired mutations (such as those caused by diet, environment, and/or lifestyle) or somatic mutations. Examples of such cancers may include, but are not limited to, adrenal cancer, adrenocortical cancer, bladder cancer, brain cancer, primary brain cancer, glioma, glioblastoma, breast cancer, cervical cancer, colon cancer (non-limiting examples include colorectal cancer, such as colon adenocarcinoma and colon adenoma), endometrial cancer, epithelial cancer, esophageal cancer, gallbladder cancer, genitourinary tract cancer, head and neck cancer, kidney cancer, liver cancer, lung cancer (non- Limiting examples include adenocarcinoma, small cell lung cancer, and non-small cell lung cancer), lymphoma (non-limiting examples include B cell lymphoma, T cell lymphoma, Hodgkin's lymphoma, non-Hodgkin's lymphoma) , melanoma, malignant melanoma, malignant carcinoid, malignant pancreatic insulinoma, myeloma, multiple myeloma, ovarian cancer, pancreatic cancer (such as exocrine pancreatic cancer), prostate cancer, renal cell carcinoma, skin cancer (except cancers other than those previously mentioned, such as squamous cell carcinoma), gastric cancer, testicular cancer, thyroid cancer, thyroid follicular carcinoma, Wilm's tumor, choriocarcinoma, mycosis fungoides, malignant hypercalcemia, Cervical hyperplasia, **leukemia,** acute lymphoblastic leukemia, chronic lymphocytic leukemia, hairy cell lymphoma, Burkitt's lymphoma, acute myeloid leukemia, chronic myelogenous leukemia, myelodysplastic syndrome, promyelocytic Leukemia, chronic myeloid leukemia, acute myeloid leukemia, fibrosarcoma, rhabdomyosarcoma, astrocytoma, neuroblastoma, rhabdomyosarcoma, schwannoma, Kaposi's sarcoma, polycythemia vera, essential platelets Polycythemia, Hodgkin's disease, non-Hodgkin's lymphoma, soft tissue sarcoma, osteosarcoma, primary macroglobulinemia, seminoma, teratoma, osteosarcoma, dry skin pigmentation keratosis, keratoacanthoma, and retinoblastoma.

"Metastatic cancer" is cancer in which cancer cells from one organ or body part have spread (by "metastasis") to another non-adjacent organ or body part. Cancer at a non-adjacent organ or body part ("secondary tumor" or "metastatic tumor") includes cancer cells originating from an organ or body part where the cancer or cancer cells have spread from that organ or body part. Sites where secondary tumors can occur include, but are not limited to, lymph nodes, lung, liver, brain, and/or bone.

"PIK3CA mutation cancer" refers to the amino acid change of PIK3CA gene in cancer cells due to nucleic acid base mutation, which eventually leads to abnormality of PI3K cell signal transmission. The mutation sites include but are not limited to H1047R, E542K and E545K.

Herein, the terms "pharmaceutically usable" and "pharmaceutically acceptable" are used interchangeably to refer to the type generally accepted by those skilled in the pharmaceutical arts. For example, pharmaceutically acceptable salts, pharmaceutically acceptable carriers and the like.

"Oral dosage form" refers to a pharmaceutical formulation prepared for use in an individual by the oral route of administration. Examples of known oral dosage forms include, but are not limited to, tablets, capsules, powders, pills, granules, suspensions, solutions and solution pre-concentrates, emulsions and emulsion pre-concentrates, and the like. In some ways, powders, pills, granules, capsules and tablets can be coated with suitable polymers or commonly used coating materials to achieve, for example, greater stability in the gastrointestinal tract or to achieve a desired rate of release. In addition, the capsule shells of powders, pills or granules are further coated. Tablets may be scored to facilitate dosing division.

When the compound of formula I or formula II according to the present invention is administered as an oral preparation, it is preferably film-coated. Suitable films are known in the art and are commercially available or can be manufactured according to known methods. Typically, the film coating material is a hydrophilic polymer such as polyethylene glycol, polyvinylpyrrolidone, polyvinyl alcohol, hydroxypropylcellulose, hydroxymethylcellulose, and hydroxypropylmethylcellulose, among others. The film coating composition ingredients may include plasticizers such as polyethylene glycol, triethyl citrate, diethyl phthalate, propylene glycol, glycerin in usual amounts; as well as opacifiers such as titanium dioxide and colorants such as oxidized iron, aluminum lakes, etc. Typically, the film coating material is applied in such an amount as to provide a film coating in the range of 1% to 6% of the solid oral dosage form, for example.

When the compound of formula I or formula II of the present invention is administered as an oral preparation, it may optionally further contain at least one pharmaceutically acceptable carrier used in medicine. "Pharmaceutically acceptable carrier" refers to any pharmaceutically inert material that is substantially inactive in biological activity and that constitutes a substantial part of the **formulation.** Examples of such carriers include, but are not limited to, diluents and fillers, disintegrants, glidants, binders, stabilizers, colorants, flavor enhancers and preservatives.

As diluents, for example but not limited to: microcrystalline cellulose, mannitol, powdered sugar, compressible sugar, dextran, dextrin, dextrose, lactose, cellulose powder, sorbitol, sucrose and talc or a combination thereof. Wherein, the diluent may be 5% to 90% based on the total weight of the oral preparation, preferably 10% to 80%, 20%-70%, 30%-60%, 40%-50%.

As disintegrants, for example but not limited to: cellulose, alginates, gums, cross-linked polymers such as crosslinked polyvinylpyrrolidone or crospovidone, croscarmellose sodium, croscarmellose calcium, soybean polysaccharide, sodium starch glycolate, guar gum, or any combination thereof.

Wherein, the diluent may be present in an amount of about 1% to 15%, preferably 2% to 10%, based on the total weight of the oral preparation.

As a binder, for example but not limited to starch, cellulose or its derivatives such as microcrystalline cellulose, hydroxypropyl cellulose, hydroxyethyl cellulose and hydroxypropyl methyl cellulose, sucrose, dextrose sugar, corn syrup, polysaccharides, gelatin, or any combination thereof. Wherein, the binder may be present in an amount of 0.01 to 10%, preferably 1%-10%, based on the total weight of the preparation.

As the glidant, for example, but not limited to, colloidal silicon dioxide, magnesium trisilicate, cellulose powder, talc or a combination thereof can be selected. Glidants may be present in amounts ranging from 0.1% to 10%, preferably from 0.1% to 0.5%, based on the total weight of the composition.

Unless otherwise stated, the terms "comprise" and "include" as used herein have an open and non-limiting meaning. For example, in addition to the pharmaceutical combination formed by the PI3K inhibitor, PGE2 receptor inhibitor and PD-1 inhibitor of the present invention, other therapeutic agents such as "biotherapeutic agents" and "chemotherapeutic agents" may also be included.

Herein, "biotherapeutic agent" refers to a biomolecule, such as an antibody or a fusion protein, that blocks ligand/receptor activity in any biological pathway that supports tumor maintenance and/or growth or suppresses tumor immune responses. For example, "biotherapeutic agents" include, but are not limited to: alemtuzumab, bevacizumab, Berentuzumab vedotin, catumaxomab, cetuximab, denosumab, gemtuzumab monoclonal antibody, ipilimumab, nimotuzumab, ofatumumab, panitumumab, rituximab, tositumomab, trastuzumab, nivolumab, atezolizumab, durvalumab, avelumab.

Among them, "chemotherapeutic agents" are chemical compounds that can be used in cancer treatment, including but not limited to alkylating agents, antimetabolites, kinase inhibitors, spindle toxin plant alkaloids, cytotoxic/antitumor biotin, topoisomerase inhibitor, photosensitizers, antiestrogens and selective estrogen receptor modulators, aromatase inhibitors, EGFR inhibitors, VEGF inhibitors, antisense oligonucleotides that inhibit the expression of genes associated with abnormal cell proliferation or tumor growth. For example, examples of chemotherapeutic agents include, but are not limited to, chlorambucil, melphalan, cyclophosphamide, ifosfamide, busulfan, carmustine, lomustine, streptozotocin, Cisplatin, carboplatin, oxaliplatin, dacarbazine, temozolomide, procarbazine, methotrexate, fluorouracil, cytarabine, gemcitabine, mercaptopurine, fludarabine, vinblastine, vincristine, Vinorelbine, paclitaxel, docetaxel, topotecan, irinotecan, etoposide, trabectedin, dactinomycin, doxorubicin, epirubicin, daunomycin, mitoxantrone , bleomycin, mitomycin C, ixabepilone, tamoxifen, flutamide, gonadorelin analogs, megestrol, prednisone, dexamethasone, methylprednisolone, Thalidomide, interferon alfa, leucovorin, sirolimus, temsirolimus, everolimus, afatinib, alisertib, amuvatinib, apatinib, axitinib, boron Tezomib, bosutinib, britinib, cabozantinib, cediranib, crenolanib, crizotinib, dabrafenib, dacomitinib, danucetib, dasatinib, dovitinib, erlotinib, foretinib, ganetespib, gefitinib, ibrutinib, icotinib, imatinib, iniparib, lapatinib, lenvatinib, linifanib, linsitinib, masitinib, momelotinib , motisanib, neratinib, nilotinib, niraparib, oprozomib, olaparib, pazopanib, pictilisib, ponatinib, quizartinib, regorafenib, rigosertib, rucaparib, ruxolitinib, saracatinib, saridegib, sorafenib, sunitinib, tiratinib, tivantinib, tivozanib, tofacitinib, trametinib, vandetanib, veliparib, Verofini, Vimodeji, volasertib.

The choice of dosage regimen for the combination therapy of the invention will depend on factors such as: the rate of substantial serum or tissue turnover, the level of symptoms, the overall immunogenicity and the accessibility of the target cells, tissues or organs in the individual being treated. Preferably, the dosage regimen maximizes the amount of each therapeutic agent delivered to the patient, consistent with acceptable levels of side effects. Thus, the dosage and frequency of administration of each biotherapeutic and chemotherapeutic agent in the combination therapy will depend in part on the particular therapeutic agent, the severity of the cancer being treated and the characteristics of the patient.

The combination therapy of the invention may be administered before or after surgery to remove the tumor, and may be administered before, during, or after radiation therapy.

WO2007084786 specifically describes the compound of formula I of the present invention, the compound or its pharmaceutically acceptable salt and its preparation process are disclosed in the examples of WO2007084786, which is incorporated herein by reference in its entirety.

WO2012039972 specifically describes the compound of formula II of the present invention, the compound or its pharmaceutically acceptable salt and its preparation process are disclosed in the examples of WO2012/039972, which is incorporated herein by reference in its entirety.

The present invention is further illustrated by the following examples, but the examples are not meant to limit the protection scope of the present invention in any way.

In some embodiments, administration of a PI3K inhibitor, an EP-4 inhibitor, and a PD-1 inhibitor in combination results in potentiation of the PD-1 inhibitor such that, for example, smaller doses or longer intervals of PD-1 inhibitor may be effective for treatment.

"Antibody" includes immunoglobulins of all classes, including IgG, IgM, IgA, IgD, and IgE, or fragments thereof, which may be suitable for the medical uses disclosed herein. Antibodies may be monoclonal or polyclonal and may be of any species origin including, for example, mouse, rat, rabbit, horse or human. Antibody fragments that retain specific binding to the protein or epitope (PD-L1 or PD-1) to which the antibody used in the present invention binds are included within the scope of the term "antibody". Such fragments can be produced by known techniques. Antibodies can be chimeric or humanized, especially when they are used for therapeutic purposes. Antibodies can be obtained or prepared using methods known in the art.

So that the invention described herein may be more fully understood, the following examples are set forth. It should be understood that these examples are for illustrative purposes only and are not to be construed as limiting the invention in any way.

### Specific Embodiments

Example 1: Combination therapy of formula I compound (AN2025), formula II compound (AN0025) and PD-1 antibody in the treatment of mouse EMT6, CT26 tumors

### Materials and methods

Reagents and instruments: AN0025 was purchased from Shanghai Caerulum Pharma Discovery Co.,Ltd.; AN2025 was purchased from CSNpharm. Antibody against **mouse** PD-1 (clone number: RMP1-14) was purchased from BioXcell.

Cell lines: Mouse colon cancer CT26 cells (CRL-2638) and breast cancer EMT6 cells (CRL-2755) were purchased from American Tissue Culture Center. All cells were cultured in RPMI-1640 (CT26) or Waymouth's (1x) MB 752/1 (EMT6) medium supplemented with 10% fetal bovine serum in an incubator at 37°C and 5% CO₂. Subculture twice a week until the necessary number of cells for inoculating **mice** is obtained.

Animals: 6-8 week old BalB/c female mice were purchased from Shanghai Slac Experimental Animal Technology Co., Ltd. Animals were housed in micro-isolator cages of up to 5 per cage on a 12-h light/dark cycle. Cages were changed twice a week. Animals were observed weekly and clinical signs were recorded.

Animal research: Harvest cancer cells cultured in vitro, suspend in 100 µl phosphate-buffered saline at a cell concentration of 2.0×105 cells (CT26)/ml and 2.5×105 cells (EMT6)/ml, and use a syringe to inject into mice subcutaneously (sc) on the dorsum of the right forelimb. 6-7 days after cell implantation, mice were randomly assigned based on tumor size, and for the EMT6 model, AN2025 was administered: 30 mg/kg, orally (PO) once a day. AN0025 administration: 150mg/kg, orally (PO) once a day. PD-1 antibody administration: 10mg/kg, intraperitoneal injection (IP) twice a week; for CT26 model, AN2025 administration: 30mg/kg, oral administration (PO) once a day. AN0025 administration: 100mg/kg, orally (PO) once a day. PD-1 antibody administration: 10mg/kg, administered by intraperitoneal injection (IP) twice a week. Animals assigned to treatment groups in each study had similar mean tumor weights in all groups. Tumor size was measured 2-3 times a week with digital calipers and volume was calculated using the formula (l × w2)/2 = mm3, where 1 and w refer to the larger and smaller orthogonal dimensions collected at each measurement. Cohort tumor size (mean ± SEM) was plotted versus time using GraphPad Prism 6 software. Statistical analysis was performed using Independent Sample t-test for analysis.

### Experimental results

As shown in Figure 1 and Figure 2, although the combination of AN0025 and PD-1 antibody and the combination of AN2025 and PD-1 antibody have shown certain anti-inflammatory effects on the mouse breast cancer EMT6 model and the mouse colorectal cancer CT26 model, the antitumor activity of combination of the three, that is, the anti-tumor activity brought by the triple combination of AN2025, AN0025 and PD-1 antibody was significantly better than that of AN0025 and PD-1 antibody double combination (p<0.05) and AN2025 and PD-1 antibody double combination (p<0.05).

### Example 2: Combination therapy of AN2025, AN0025 and PD-1 antibody in the treatment of HCT116 tumors in PBMC immune system humanized mice

### Materials and methods

Reagents and instruments: AN0025 was purchased from Shanghai Caerulum Pharma Discovery Co.,Ltd.; AN2025 was purchased from CSNpharm. Antibody against human PD-1 (Pembrolizumab) was provided by Phenotek Biotechnology Co., Ltd. Human PBMCs were purchased from Milestone (Shanghai) Biotechnology Co., Ltd. Cell line: HCT116 human colon carcinoma cells were purchased from the Cell Bank of the Chinese Academy of Sciences. Cells were cultured in McCoy's 5a medium supplemented with 10% fetal bovine serum in an incubator at 37°C and 5% CO₂, and subcultured twice a week until the necessary number of cells were obtained for inoculation of mice .

Animals: 6-8 week old NPSG female mice were purchased from PNK. Animals were housed in IVC isolation cages, 5 per cage, with a 12-h light/dark cycle. Temperature 20~26°C, humidity 30-70%.

Animal research: NPSG mice were injected IV with the inoculation amount of human PBMC 5×106/100ul. Six days after inoculation, HCT116 cells were subcutaneously inoculated on the right shoulder of the **mice** at a dose of 1×106/mice. After inoculation, the tumor volume and body weight were measured once a week, and when the average tumor volume reached about 80-100mm3, the proportion of hCD45+ cells in the mouse blood was measured. According to the tumor volume and the proportion of hCD45+ cells in the mouse blood, the mice were randomly divided into 6 groups, 10 in each group. When grouping, the coefficient of variation of tumor volume CV should be ≤30% (CV=standard deviation/mean value). **Administration** was started immediately after grouping. AN2025 administration: 37.5mg/kg, orally (PO) once a day. AN0025 administration: 30mg/kg, orally (PO) once a day. PD-1 antibody administration: 10mg/kg, intraperitoneal injection (IP) twice a week. After the **administration** started, the body weight and tumor volume of the mice were measured twice a week. The proportion of hCD45+ cells in the mouse blood was monitored by flow cytometry once a week. Animals assigned to treatment groups in each study had similar mean tumor weights in all groups. Tumor size was calculated using the formula (l × w2)/2 = mm3, where 1 and w refer to the larger and smaller orthogonal dimensions collected in each measurement. For pairwise comparisons, the T-Test analysis method was used, and for comparisons between more than three groups, one-way analysis of variance (One-Way ANOVA) was used for analysis. For comparison of potential synergistic effects, two-way analysis of variance (Two-Way ANOVA) was used. All data use SPSS 24.0. A p value less than 0.05 was considered to be significantly different.

### Experimental results

Although the combination of AN0025 and PD-1 antibody and the combination of AN2025 and PD-1 antibody showed certain anti-tumor activity in HCT116 tumors of PBMC immune system humanized mice, the anti-tumor activity brought by the combination of the three, namely AN2025, AN0025 and PD-1 antibody triple combination was significantly better than the activity of AN0025 and PD-1 antibody double combination (p<0.05) and the activity of AN2025 and PD-1 antibody double combination (p<0.01).

## Claims

1. A method for treating tumor/cancer and/or generating a memory immune response against tumor/cancer in an object in need thereof, comprising administering an effective amount of PI3K inhibitors, prostaglandin receptors (PGE2) inhibitors and PD-1 inhibitors to said object, wherein the PI3K inhibitors, prostaglandin receptor (PGE2) inhibitors and the PD-1 inhibitors can be administered simultaneously, separately or sequentially as a single dosage form.

2. The method of claim 1, wherein the PI3K inhibitor is selected from PI3Kα, PI3Kβ, PI3Kγ, PI3Kδ subtype inhibitors.

3. The method according to claim 1 or 2, wherein the PI3K inhibitor is selected from Idelalisib, Copanlisib, Duvelisib, Alpelisib, Seletalisib, Gedatolisib, Rigosertib sodium, Leniolisib, Umbralisib, Buparlisib (AN2025), AMG-319, GM -604, Acalisib, Bimiralisib, GDC-0084, ACP-319, Tenalisib, serabelisib, SF-1126, Nemiralisib, Fimepinostat, LY-3023414, Voxtalisib, Dactolisib, Parsaclisib, GSK-2636771, AZD-8186, ASN-003 and any combination thereof.

4. The method according to any one of claims 1-3, wherein the PI3K inhibitor is a compound of formula (I) or a pharmaceutically acceptable salt thereof,

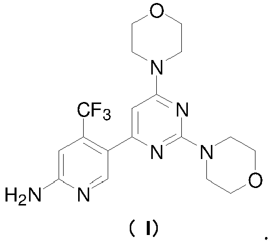

5. The method of any one of claims 1-4, wherein the prostaglandin receptor (PGE2) inhibitors include EP-1, EP-2, EP-3 and EP-4 inhibitors.

6. The method according to any one of claims 1-5, wherein the prostaglandin receptor (PGE2) is selected from compounds having formula (II) or pharmaceutically acceptable salts thereof.

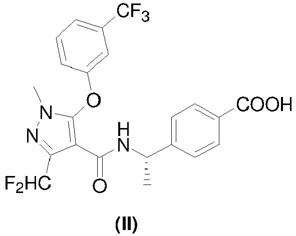

7. The method of any one of claims 1-6, wherein the PD-1 inhibitor comprises PD-1/PDL-1 antibody therapy.

8. The method according to any one of claims 1-7, wherein the PD-1 inhibitor is selected from: PD-1 antibody, PD-L1 antibody and PD-L2 antibody; preferably, wherein the PD-1 inhibitor is selected from Nivolumab, Pembrolizumab, Atezolizumab, Avelumab, Durvalumab, Tremelimumab, Toripalimab, Sintilimumab, Tislelizumab, Camrelizumab and any combination thereof.

9. The method according to any one of claims 1-8, wherein the PI3K inhibitor is administered via oral, buccal or parenteral route, for example, the dosage form of the oral route can be tablets, capsules, powders, pills, granules, suspensions, solutions and solution preconcentrates, emulsions and emulsion preconcentrates, for example, the dosage form of the parenteral route can be intravenous, intraperitoneal, intradermal, subcutaneous, intramuscular, intracranial, Intrathecal, intratumoral, transdermal, transmucosal administration.

10. The method according to any one of claims 1-9, wherein the PI3K inhibitor is administered once or twice a day; or the PI3K inhibitor is administere once every 2, 3, 4, 5, 6, 7, 8, 9 or 10 days or every 1, 2 or 3 weeks; or the PI3K inhibitor is administered once a day for 5 consecutive days a week, followed by an interval of 2 days.

11. The method according to any one of claims 1-10, wherein the PI3K inhibitor is administered in adults at a dose range of from about 20 mg/day-about 200mg/day, about 30mg/day-about 160mg/day, about 60mg/day-about 120 mg/day.

12. The method according to any one of claims 1-11, wherein the PI3K inhibitor is administered to the subject at an effective dose of about 0.5 to about 250 mg/kg, 1 to about 250 mg/kg, about 2 to about 200 mg/kg, about 3 to about 120 mg/kg, about 5 to about 250 mg/kg, about 10 to about 200 mg/kg, or about 20 to about 120 mg/kg.

13. The method according to any one of claims 1-12, wherein the prostaglandin receptor (PGE2) inhibitor is administered in a dosage form of oral, buccal or parenteral routes, such as the dosage form of the oral route can be tablets, capsules, powders, pills, granules, suspensions, solutions and solution preconcentrates, emulsions and emulsion preconcentrates, for example the dosage form for the parenteral route can be intravenous, intraperitoneal, intradermal, subcutaneous , intramuscular, intracranial, intrathecal, intratumoral, transdermal, transmucosal administration.

14. The method according to any one of claims 1-13, wherein the prostaglandin receptor (PGE2) inhibitor is administered once or twice every day; or the prostaglandin receptor (PGE2) inhibitor is administered once every 2, 3, 4, 5, 6, 7, 8, 9, or 10 days or every 1, 2, or 3 weeks; or the prostaglandin receptor (PGE2) inhibitor is administered once daily for 5 consecutive days per week, followed by an interval of 2 days.

15. The method according to any one of claims 1-14, wherein the prostaglandin receptor (PGE2) is administered in adults at a dose range of about 20 mg/day to about 2000mg/day, about 30mg/day to about 1600mg/day , about 60 mg/day to about 1200 mg/day or about 100 mg/day to about 1000 mg/day.

16. The method according to any one of claims 1-15, wherein the prostaglandin receptor (PGE2) inhibitor is administered to the subject at an effective dose of about 0.5 to about 250 mg/kg, about 1 to about 250 mg/kg, about 2 to about 200 mg/kg, about 3 to about 120 mg/kg, about 5 to about 250 mg/kg, about 10 to about 200 mg/kg, or about 20 to about 120 mg/kg.

17. The method according to any one of claims 1-16, wherein the PD-1 inhibitor is administered by a parenteral route, for example, the parenteral route can be intravenous, intraperitoneal, intradermal, subcutaneous, intramuscular, intracranial, intrathecal, intratumoral, transdermal, transmucosal administration.

18. The method according to any one of claims 1-17, wherein the PD-1 inhibitor is formulated into a solution, a lyophilized agent, or a powder injection.

19. The method according to any one of claims 1-18, wherein the PD-1 inhibitor is administered at an effective dose of 0.05mg/kg, 0.1mg/kg, 1mg/kg, 2mg/kg, 3mg/kg, 4mg/kg, 5mg/kg, 6mg/kg, 7 mg/kg or 8 mg/kg.

20. The method according to any one of claims 1-19, wherein the tumor/cancer is selected from PIK3CA mutated cancers.

21. The method of any one of claims 1-20, wherein the tumor/cancer is selected from the group consisting of: head and neck squamous cell carcinoma, head and neck cancer, brain cancer, glioma, glioblastoma multiforme, neuroblastoma, central nervous system cancer, neuroendocrine tumor, throat cancer, nasopharyngeal cancer, esophageal cancer, thyroid cancer, malignant pleural mesothelioma, lung cancer, breast cancer, liver cancer, hepatoma, hepatobiliary cancer, pancreatic cancer, gastric cancer, gastrointestinal cancer, bowel cancer, colon cancer, colorectal cancer, kidney cancer, clear cell renal cell carcinoma, ovarian cancer, endometrial cancer, cervical cancer, bladder cancer, prostate cancer, testicular cancer Carcinoma, skin cancer, melanoma, leukemia, lymphoma, bone cancer, chondrosarcoma, myeloma, multiple myeloma, myelodysplastic syndrome, myeloproliferative neoplasm, squamous cell carcinoma, Ewing's sarcoma, systemic Light chain amyloidosis and Merkel cell carcinoma;
more preferably, said lymphoma is selected from: Hodgkin's lymphoma, non-Hodgkin's lymphoma, diffuse large B-cell lymphoma, follicular lymphoma, primary mediastinal large B-cell lymphoma, mantle cell lymphoma, small lymphocytic lymphoma, T-cell/histiocytic-rich large B-cell lymphoma, and lymphoplasmacytic lymphoma; said lung cancer is selected from: non-small cell lung cancer and small cell lung cancer; said leukemia is selected from: chronic myeloid leukemia, acute myeloid leukemia, lymphocytic leukemia, lymphoblastic leukemia, acute lymphoblastic leukemia, chronic lymphocytic leukemia leukemia and myeloid leukemia.

22. A pharmaceutical composition and/or pharmaceutical combination, which comprises a PI3K inhibitor, a prostaglandin receptor (PGE2) inhibitor and a PD-1 inhibitor, and a pharmaceutically acceptable carrier.

23. The pharmaceutical composition and/or pharmaceutical combination according to claim 22, wherein the PI3K inhibitor is selected from PI3Kα, PI3Kβ, PI3Kγ, PI3Kδ subtype inhibitors.

24. The pharmaceutical composition and/or pharmaceutical combination according to claim 22 or 23, wherein said PI3K inhibitor is selected from Idelalisib, Copanlisib, Duvelisib, Alpelisib, Seletalisib, Gedatolisib, Rigosertib sodium, Leniolisib, Umbralisib, Buparlisib (AN2025 ), AMG-319, GM-604, Acalisib, Bimiralisib, GDC-0084, ACP-319, Tenalisib, serabelisib, SF-1126, Nemiralisib, Fimepinostat, LY-3023414, Voxtalisib, Dactolisib, Parsaclisib, GSK-2636771, AZD- 8186, ASN-003 or any combination thereof.

25. The pharmaceutical composition and/or pharmaceutical combination according to any one of claims 22-24, wherein the PI3K inhibitor is a compound of formula (I) or a pharmaceutically acceptable salt thereof,

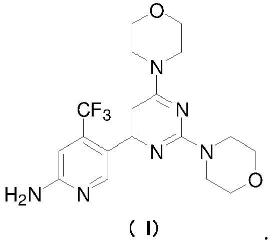

26. The pharmaceutical composition and/or pharmaceutical combination according to any one of claims 22-25, wherein said prostaglandin receptor (PGE2) inhibitor is selected from EP-1, EP-2, EP-3 and EP-4 inhibitors.

27. The pharmaceutical composition and/or pharmaceutical combination according to any one of claims 22-26, wherein the EP-4 inhibitor is a compound of formula (II) or a pharmaceutically acceptable salt thereof.

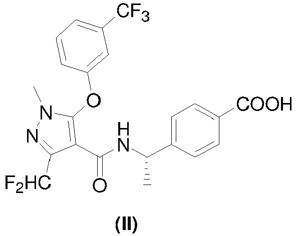

28. The pharmaceutical composition and/or pharmaceutical combination according to any one of claims 22-27, wherein the PD-1 inhibitor is selected from: PD-1 antibody, PD-L1 antibody and PD-L2 antibody; preferably, wherein the PD-1 inhibitor is selected from Nivolumab, Pembrolizumab, Atezolizumab, Avelumab, Durvalumab, Tremelimumab, Toripalimab, Sintilimumab, Tislelizumab, Camrelizumab or any combination thereof.

29. The pharmaceutical composition and/or pharmaceutical combination according to any one of claims 22-28, wherein the PI3K inhibitor, prostaglandin receptor (PGE2) inhibitor and PD-1 inhibitor can be in the same and/or or in separate dosage forms.

30. The pharmaceutical composition and/or pharmaceutical combination according to any one of claims 22-29, wherein the PI3K inhibitor can be formulated into a dosage form via oral, buccal, or parenteral route, for example the dosage forms for the oral route may be tablets, capsules, powders, pills, granules, suspensions, solutions and solution preconcentrates, emulsions and emulsion preconcentrates, for example the dosage forms for the parenteral route may be intravenous, intraperitoneal, intradermal, subcutaneous, intramuscular, intracranial, intrathecal, intratumoral, transdermal, transmucosal administration, and the dosage form can be, for example, solution, powder injection or lyophilized preparation.

31. The pharmaceutical composition and/or pharmaceutical combination according to any one of claims 22-30, wherein the prostaglandin receptor (PGE2) inhibitor can be formulated to be dosage forms administered via oral, buccal or parenteral route, for example, the dosage form of the oral route can be tablets, capsules, powders, pills, granules, suspensions, solutions and solution preconcentrates, emulsions and emulsion preconcentrates, for example, the dosage form of the parenteral route can be intravenous, intraperitoneal, intradermal, subcutaneous, intramuscular, intracranial, intrathecal, intratumoral, transdermal, transmucosal administration, and the dosage form can be, for example, solutions, powder injections or lyophilized formulations.

32. The pharmaceutical composition and/or pharmaceutical combination according to any one of claims 22-31, wherein the PD-1 inhibitor can be formulated into a dosage form administered by an parenteral route, for example, the parenteral route can be intravenous, intraperitoneal, intradermal, subcutaneous, intramuscular, intracranial, intrathecal, intratumoral, transdermal, transmucosal administration, and the dosage form, for example, may be a solution, powder injection or lyophilized preparation.

33. The pharmaceutical composition and/or pharmaceutical combination according to any one of claims 22-32, wherein each unit dosage form contains PI3K inhibitor in a dose of 1-1000mg, such as 1, 2, 3, 4, 5, 6, 7, 8, 9, 10, 11, 12, 13, 14, 15, 16, 17, 18, 19, 20, 25, 30, 35, 40, 50, 60, 70, 75, 80, 90, 100, 110, 120, 125, 130, 140, 150, 160, 170, 175, 180, 190, 200, 250, 300, 350, 400, 450, 500, 600, 700, 750, 800, 900, 1000mg or a value between any two values above.

34. The pharmaceutical composition and/or pharmaceutical combination according to any one of claims 22-33, wherein each unit dosage form contains the prostaglandin receptor (PGE2) inhibitor in a dose of 1-1000mg, such as 1, 2, 3, 4, 5, 6, 7, 8, 9, 10, 11, 12, 13, 14, 15, 16, 17, 18, 19, 20, 25, 30, 35, 40, 50, 60, 70, 75, 80, 90, 100, 110, 120, 125, 130, 140, 150, 160, 170, 175, 180, 190, 200, 250, 300, 350, 400, 450, 500, 600, 700, 750, 800, 900, 1000 mg or a value between any two values above.

35. The pharmaceutical composition and/or pharmaceutical combination according to any one of claims 22-34, wherein each unit dosage form contains the PD-1 inhibitor in a dose of 1-5000 mg, such as 1, 2, 3, 4, 5 , 6, 7, 8, 9, 10, 11, 12, 13, 14, 15, 16, 17, 18, 19, 20, 25, 30, 35, 40, 50, 60, 70, 75, 80, 90,100,110,120,125,130,140,150,160,170,175,180,190,200,250,300,350,400,450,500, 600,700,750,800,900,1000 ,1100,1200,1300,1400,1500,1600,1700,1800,1900,2000,2 200,2400,2500,2600,2700,2750,2800,3000,3500,4000,4500,5000mg or a value between any two values above.

36. The pharmaceutical composition and/or pharmaceutical combination according to any one of claims 22-35, which is used for treating tumor/cancer and/or generating memory immune response against tumor/cancer.

37. Use of the pharmaceutical composition and/or pharmaceutical combination according to any one of claims 22-35 for preparing a medicine for treating tumor/cancer and/or generating memory immune response against tumor/cancer.

38. A kit, comprising the pharmaceutical composition and/or pharmaceutical combination according to any one of claims 22-35, and instructions for use, wherein the PI3K inhibitor, prostaglandin receptor (PGE2) inhibitor and PD-1 inhibitors can be in the same and/or separate containers
